# EUROPEAN PATENT APPLICATION

(11) **EP 4 235 685 A1**
(43) Date of publication of application: **30.08.2023**
(21) Application number: 22158204.2
(22) Date of filing: 23.02.2022
(51) Int. Cl.: G16H 30/00, G16H 50/20, G06N 3/02

(54) **METHOD, SYSTEM AND COMPUTER PROGRAM FOR DETECTION OF A DISEASE INFORMATION**

(71) Applicant: Siemens Healthcare GmbH, 91052 Erlangen (DE); Friedrich-Alexander-Universität Erlangen-Nürnberg, 91054 Erlangen (DE)
(72) Inventor: Thamm, Florian, 90762 Fürth (DE); Jürgens, Markus, 91325 Adelsdorf (DE); Taubmann, Oliver, 91365 Weilersbach (DE); Ditt, Hendrik, 91413 Neustadt an der Aisch (DE)

(57) **Abstract**

A method for generating training data (1) for training a deep learning algorithm (6), comprising:
- Receiving (RT1) medical imaging data (2) of an examination area of a patient, the examination area of the patient comprising a first and a second part of a symmetric organ,
- Splitting (SP1) the medical imaging data (2) along a symmetry plane or symmetry axis into a first dataset (3a) and a second dataset (3b), wherein the first dataset (3a) comprises the medical imaging data (2) of the first part of the organ and the second dataset comprises the medical imaging data (2) of the second part of the organ,
- Mirroring (MD1) the second dataset (3b) along the symmetry plane or symmetry axis,
- Generating (GT1) the training data (1) by stacking of the first dataset (3a) and the mirrored second dataset (3b*)
- Providing (PT1) the training data (1).

## Description

Embodiments of the invention generally relate to a generation of training data for a deep learning algorithm to detect a disease information, especially for a classification, localization and/or characterization of disease information. The disease information is for example a cerebral disease information for a cerebral disease e.g. a large vessel occlusion. Embodiments of the invention further generally relate to a method for training a deep learning algorithm to detect a disease information with training data, a method to detect disease information and to a computer program.

The invention generally concerns the field of medical imaging and medical image analysis. Medical imaging can be performed with a variety of imaging modalities such as X-ray, ultrasound, magnetic resonance imaging (MRI), computed medical (CT), PET, SPECT etc. as are known in the art. The imaging is performed with a respective imaging device, also referred to as scanner. In particular, the invention is in the field of the detection of cerebrovascular diseases by medical imaging. The invention is especially for detecting and/or providing information for cerebrovascular diseases like a stroke and/or a large vessel occlusion.

Stroke remains one of the leading causes of death in developed countries. Nearly nine out of ten strokes are ischemic, i.e., they are caused by a thrombus (blood clot) occluding a vessel, thereby preventing part of the brain from being supplied with oxygenated blood. Mechanical thrombectomy (MT), the physical removal of thrombi, became one of the key treatments in stroke patients with acute cerebral ischemia caused by large vessel occlusions (LVO).

Typically, stroke patients first undergo a non-contrast computed tomography (CT) head scan for initial assessment. In case of an ischemic stroke, it is essential to quickly identify which parts of the brain are affected in order to choose the most promising treatment. However, this is a challenging task as the early signs of ischemia in non-contrast scans are often subtle. A CT angiography (CTA) scan is usually performed to reliably determine the affected regions, assess the collateral status and localize the thrombus. Supporting experts with their diagnosis by the automated software-based detection and localization can save time and can save time to help for earlier re-canalization.

In particular, it is differentiated between the classification and the localization of a disease information, especially of cerebrovascular disease and/or LVOs. The classification and localization are often summarized as detection. Particularly, detection, classification and/or localization of the disease information means detection, classification and/or localization of the disease, wherein the disease information is for example an anatomic, physical and/or tissue anomaly caused by the disease. In other words, the disease information is configured, results in and/or comprises a radiological finding. The classification is the task of determining, whether a patient is suffering a disease, especially a cerebral disease. This is leading to a two-class problem (e.g., LVO positive, LVO negative). This can be extended to a three-class problem, wherein the side is predicted as well (Left LVO, Right LVO, No LVO). An advancement of the classification is the localization. Here the task is to determine the coordinate of the disease information and/or of the LVO in the patients' coordinate system. The localization is a regression task. The localization is much more desirable, as this leads to the final piece of information to proceed with the treatment of the patient. Furthermore, after localization the radiologist can easily confirm the classification results.

The automated detection and localization of diseases Information, especially of LVOs, is facing several challenges. The anatomy of organs, especially of the cerebrovascular vessel tree, is highly individual. Consequently, for approaches based on trainable methods, for instance deep learning based techniques, a high amount of data is required to achieve an acceptable level of generalization. Especially, the different amount of contrast agent injected is another degree of freedom in scans. Furthermore, comparing the left and right hemisphere is of crucial importance in the detection of cerebral disease information, especially of LVOs. Yet it is difficult to integrate prior knowledge in trainable architectures for the detection of cerebral disease information.

The underlying technical problem of the invention is to improve the automated detection, classification and/or localization of disease information based on a deep learning algorithm. Furthermore, a technical problem of the invention is to provide the trained deep learning algorithm and, especially, to provide training data for the training. The problems are solved by the subject matter of the independent claims. The dependent claims are related to further aspects of the invention.

In the following the solution according to the invention is described with respect to the claimed methods as well as with respect to the claimed computer program and system. Features, advantages or alternative embodiments herein can be assigned to the other claimed objects and vice versa. In other words, claims for the method can be improved with features described or claimed in the context of the computer program and system. In this case, the functional features of the method are embodied by objective units of the system. Furthermore, features of the different methods (generating training data, training of the deep learning algorithm and detection of the cerebral disease information) can be interpreted based on the features and description of any of the methods. Especially, any of the different methods can be improved with features of any other of the methods.

In one aspect the invention relates to a method for generating training data for training a deep learning algorithm, comprising:
- Receiving medical imaging data of an examination area of a patient, the examination area of the patient comprising a first and a second part of a symmetric organ,
- Splitting of the medical imaging data along a symmetry plane or symmetry axis into a first dataset and a second dataset, wherein the first dataset comprises the medical imaging data of the first part of the organ and the second dataset comprises the medical imaging data of the second part of the organ,
- Mirroring of the second dataset along the symmetry plane or symmetry axis,
- Generating of the training data by stacking of the first dataset and the mirrored second dataset,
- Providing the training data.

The medical imaging data are for example tomography imaging data. The medical imaging data can comprise computed tomography imaging data and/or magnetic resonance imaging data. Preferably the medical imaging data comprise at least one non-contrast computed tomography imaging data and/or computed tomography angiography (CTA) data, in particular digital subtraction computed tomography angiography data. The CTA data can be related to a single phase or to multiple phases. The medical imaging data can further comprise computed tomography perfusion data and/or spectral computed tomography imaging data. Spectral computed tomography imaging data can be based, for example, on dual-energy, in particular, dual-source, computed tomography imaging and/or photon-counting computed tomography imaging.

The examination area comprises at least a body part comprising the symmetric organ. The symmetric organ is for example a brain, kidneys, a lung, a female breast or a male breast. The symmetric organ comprises a first part and a second part, wherein the first and the second part are preferably two halves of the organ. In particular, the first and the second part of the organ are symmetric to the symmetry plane. The symmetric organ is for example a brain, wherein the first part and the second part are the two hemispheres of the brain. They symmetric organ can be two kidneys of a person, wherein the first part is one kidney, and the second part is the other kidney. For a lung as the symmetric organ, each part of the two parts is a lobe of the lung. The symmetry plane is especially a sagittal plane. The symmetry plane can be a hyperplane, for example a curved or displaced plane. The symmetry plane can be chosen based on an atlas of the organ. Alternatively, the symmetry plane can be chosen free or independent of an atlas. Particularly, the first part is a left part and/or a left cerebral hemisphere, and the second part is a right part and/or a right cerebral hemisphere. Alternatively, the first part is the right part, and the second part is the left part. Data, images and/or models of the second part can be transferred into data, images and/or models appearing as, optically looking like and/or modelling a first part, e.g., by applying a transformation, especially applying a geometric based transformation. Preferably, the transformation is based on mirroring, flipping, rotating, elastic deformation, augmentation and/or test time augmentation. For example, the data or images of the second cerebral hemisphere, e.g. the right cerebral hemisphere, can be mirrored on the sagittal plane, whereby the mirrored data and/or images appears like data for a first (left) cerebral hemisphere.

A symmetric organ and three-dimensional image data of such a symmetric organ have a high degree of symmetry with respect to a certain plane, the symmetry plane. Certain two-dimensional images or slices of the symmetric organ have a high degree of symmetry with respect to a symmetry axis. The degree of symmetry may differ for different two-dimensional images or slices of the symmetric organ. The symmetry plane may be defined by the plane which results in the highest degree of symmetry of the two parts of the symmetric organ or three-dimensional image data of such symmetric organ. The symmetry axis may be defined by the axis which results in the highest degree of symmetry of the two parts of the symmetric organ or the two-dimensional images or slices of such symmetric organ. Especially, paired organs are understood as symmetric organs. Symmetric organs are for example parotid gland, tonsils, nasal cavity, lungs, pleura, bones of extremities, ribs and clavicles, coccyx and pubis, pelvic bones, skin of eyelid, including canthus, skin of ear and external auditory canal, including shoulder and hip, breast, ovary, fallopian tubes, testis, epididymis, kidneys, renal pelvis, ureters, eyes, adrenals.

Preferably, the method comprises receiving of organ atlas data. The organ atlas data are for example a brain atlas data. The method may also comprise generating of registered imaging data based on the medical imaging data and the organ atlas data. The organ atlas data are used for registration. The organ atlas data can be a probabilistic brain atlas data. The generating of the registered imaging data can comprise a segmenting of the plurality of organ regions, especially brain regions, based on the medical imaging data, thereby generating, for each region of the plurality of regions, a representation of that region. The generating of the registered imaging data can comprise a scaling of the medical imaging data and/or a rigid pre-registration of the medical imaging data. For embodiments comprising receiving at least one channel (e.g. different scanning principles) and/or comprising the calculation of a channel based on the medical imaging data, the channel data comprised by the channel are preferably also registered based on the brain atlas data. The registration is especially used for registering the medical imaging data and/or the channel data into a reference coordinate system and to standardize the imaging data to a canonical pose.

The medical imaging data are split into a first dataset and a second dataset. In a preferred embodiment, for splitting of the medical imaging data the registered imaging data are used. The medical imaging data may comprise a three-dimensional image and/or model, wherein the three-dimensional image and/or model is preferably split into two three-dimensional images and/or models. Especially, the medical imaging, in particular the registered imaging data, are split and/or separated according to sagittal plane and/or a symmetry plane. Preferably, medical imaging, in particular the registered imaging data, are split into registered medical imaging data, in particular registered imaging data, data of the first and of the second part of the organ, e.g. data of the first and the second cerebral hemisphere. The first dataset comprises the medical imaging data, in particular the registered imaging data, of the first part of the organ and/or information generated based on the medical imaging data, in particular the registered imaging data, of the first part of the organ, wherein the second dataset comprises the medical imaging data, in particular the registered imaging data, of the second part of the organ and/or information generated based on the medical imaging data, in particular the registered imaging data, of the second part of the organ.

The second dataset is mirrored into the mirrored second dataset. The mirroring is especially a left-right mirroring. The second dataset is based on the second part of the organ and/or representing the second part of the organ. The second dataset is preferably mirrored in such way that the mirrored second dataset represents and/or appears as a dataset for a first part of the organ. Representing and/or appearing as a first part of the organ means especially to optically and/or geometrically look like a first part of the organ. For example, the second dataset comprises and/or describes three-dimensional model and/or images of the second cerebral hemisphere, wherein the mirrored second dataset is generated by mirroring the three-dimensional model and/or images on the sagittal plane, wherein the mirrored three-dimensional model and/or images represents and/or appears as a three-dimensional model and/or images of a first cerebral hemisphere. The mirroring of the second dataset can comprise transformations like flipping, rotating, elastic deformation, augmentation and/or test time augmentation.

The training data are generated by stacking the first dataset and the mirrored dataset together. Preferably, the training data are configured as a data package comprising the first dataset and the mirrored second dataset. The training data may have a fixed and/or standardized structure, layout and/or setup, e.g., having a stack direction. The stack direction is for example a read-out direction for the training data. Preferably, the training data are configured as a set, vector or tensor. The training data of especially configured as a tensor D in the form D={X1, X2*}, comprising the first dataset X1 and the mirrored second dataset X2*. In other words, the training data are configured as a set of data appearing and/or representing only first parts of the organ, e.g. first cerebral hemispheres. The training data are examples to train and/or learn the deep learning algorithm. In particular, in the generation of the training data the mirrored second dataset can be used as a first dataset in the training data and/or the first dataset can be used as a mirrored second dataset in the training data. For example, the training data can be generated as the tensor D=(X2*, X1).

Regarding the deep learning algorithm, it preferably is or comprises a deep convolutional neural network (DCNN). The term deep learning algorithm is according to the invention also understood as any machine learning algorithm, trained algorithm and/or algorithm based on transfer learning. The deep learning algorithm generally uses input data, especially subject patient image, to detect the disease information, especially to determine output information comprising the determination and/or detection of the disease information. The output information may comprise a classification, location and/or additional information of the disease and/or of the disease information. The additional information can comprise a size, shape, type of disease, anatomic, tissue and/or physical property. For example, in the case of LVOs, the output information may comprise a position (e.g., left, right) and/or size of the LVO. The deep learning algorithm may be an algorithm for computer aided/assisted diagnosis (CAD). The output information, thus, may only serve as a decision support and not be a diagnosis itself.

The training data are provided. Providing is for example implemented as outputting the data via a dedicated interface, a display, a printer, a memory, a network or in any other fashion suitable for inspection by a user, storage or further processing.

In summary, the invention provides training data, which are based on medical imaging data. Hence, the current invention allows to provide training data for a deep learning algorithm for the detection of disease information. In other words, the invention provides training data based on medical imaging data of two parts of a symmetric organ, especially of two cerebral hemispheres, wherein in the training data all data and/or datasets appear as, optically look like and/or describe parts of the organ, especially cerebral hemispheres, of one kind (side). This allows that medical imaging data of a second hemisphere can be mirrored into the mirrored second dataset, wherein such mirrored second datasets can be used in the training data as a first dataset or as actual mirrored second dataset. Hence the, the invention allows to mix and/or combine datasets of first and second parts of a symmetric organ, especially of two cerebral hemispheres, of different samples and/or patients. This allows the generation of more training data based on a given set of medical imaging data.

Particularly, at least one channel comprising channel data is received and/or at least one channel comprising channel data is computed based on the medical imaging data. As a channel a different scan, for example multispectral CT scan, a CTA scan or a MRI scan, can be received. Furthermore, based on the medical imaging data a segmentation of a structure or information regarding anatomic, organic and/or physical features can be computed as the channel. Particularly, at least one channel is based on windowing, especially windowing applied and/or based on the medical imaging data. For the channel, particularly, the channel data are registered based on the organ atlas data. In case of a plurality of channels, the channel data of the channels are preferably registered channel wise. The unregistered channel data or registered channel data of the channel are split into a first channel dataset and a second channel dataset. In case of a plurality of channels a plurality of first channel datasets and a plurality of second channel datasets are generated. The splitting of the channel data is especially a splitting, e.g., separation and/or portioning, along the symmetry plane, especially sagittal plane, or the symmetry axis. Hence, the unregistered or registered channel data are partitioned according to the parts of the symmetric organ, especially to the cerebral hemispheres. The first channel dataset comprises the unregistered or registered channel data of the first part of the organ, wherein the second channel dataset comprises the unregistered or registered channel data of the second part of the organ. The second channel dataset is mirrored along the symmetry plane, e.g., the sagittal plane, or along the symmetry axis. The mirroring is especially adapted and/or configured as the mirroring described for the mirroring of the second dataset. The mirrored second channel dataset appear, optically look like and/or describe channel data of a first part of the organ. The first dataset, the first channel datasets, the mirrored second dataset and the mirrored second channel dataset are stacked together as the training data. Hence, the training data comprise the first dataset, the first channel datasets, the mirrored second dataset and the mirrored second channel datasets. This embodiment generates training data including datasets for at least one additional channel, wherein all the datasets appear as datasets for the first part of the organ.

In particular, a vessel channel comprising vessel data is computed or received, wherein the vessel data are based on a segmentation of a cerebrovascular vessel tree in the medical imaging data. The vessel channel is for example computed using the method described by Thamm et al.: VirtualDSA++: Automated Segmentation, Vessel Labeling, Occlusion Detection and Graph Search on CT-Angiography Data (Tn VCBM (pp. 151-155)). Additionally and/or alternatively, a bone channel comprising bone data is computed or received, wherein the bone data are for example computed using the method described by Chen et al.: Deep learning based bone removal in computed tomography angiography (US2018116620A1). Optionally other channels might be used as well, like a non-contrast CT scan.

The training data are preferably configured as an ordered set. The ordered set can be implemented as a package, a vector or a tensor. The ordered set has for example an ordering direction, e.g., the order in which datasets will be read out by the deep learning algorithm. In the training data datasets are ordered and/or arranged such that the first dataset is prior to the first channel dataset, especially, such that the first channel dataset is prior to the mirrored second dataset and such that the mirrored second dataset is prior to the mirrored second channel datasets. When describing the structure of the training data and/or the position of the datasets in the training data the phrases first (channel) dataset and/or mirrored second (channel) dataset does not necessarily imply their origin of the part of the organ, e.g. their origin of the hemisphere. E.g., in the training data the ordering place or tensor entry of a first (channel) dataset may comprise as the first (channel) dataset an actual mirrored second (channel) dataset.

In case the datasets comprise a collection of cross section images, it is preferred that the arrangement in the training data is sectioned plane wise, e.g. {E¹(z₀), E_{c}¹(z₀), E^{2*}(z₀), E_{c}^{2*}(z₀), E¹(z₁), E_{c}¹(z₁), E^{2*}(z₁), E_{c}^{2*}(z₁),,...} wherein Eₐ^{b}(z) denotes the section image at the depth z of the dataset b, wherein a denotes the channel and * denotes mirrored.

In particular, the first dataset, the first channel datasets, the mirrored second dataset and/or the mirrored second channel datasets comprise a three-dimensional imaging dataset of the related part of the organ, e.g. the cerebral hemisphere. In case the datasets comprise the three-dimensional image datasets, it is preferred that the arrangement in the training data is dataset wise, e.g. {V¹, V_{c}¹, V^{2*}, V_{c}^{2*}} wherein Vₐ^{b} denotes the three-dimensional image dataset b, wherein a denotes the channel and * denotes mirrored.

To increase the number of achievable training data based on a limited number of medical imaging data it is preferred to stack first datasets and mirrored second datasets of different medical imaging data, especially of different patients. Alternatively or additionally, the training data can be a stack of first data sets and mirrored datasets of the same medical imaging data, especially of the same patient. Since all datasets appear as datasets for first parts of the organ, datasets can be mixed up and recombined. To increase the achievable number of training data, the idea is to mix first and second parts of the organ from different patients. Thus, for N medical imaging data, N x N combinations of first and second parts become available for training. For example, to generate training data the hemispheres are randomly stacked together, preferably considering whether they have the cerebral disease, the cerebral disease information, especially an LVO, or not. By recombination, a vast amount of data sets can be constructed.

For example, medical imaging data of three patients are received. Patient A is healthy. Patient B has a right LVO. Patient C has a left LVO. "Left" is abbreviated with "L." "Right" with "R." respectively. In terms of the hemispheres that means:
- Patient A (No LVO): L. Hemisphere healthy; R. Hemisphere healthy
- Patient B (R. LVO): L. Hemisphere healthy; R. Hemisphere LVO
- Patient C (L. LVO): L. Hemisphere LVO; R. Hemisphere healthy

These six hemispheres can now be recombined to training data. The stacking of two hemispheres is shown with a "+"-sign. The hemisphere on the left of the "+"-sign represent the left hemisphere in the stack.
- L. Hemisphere of A. (healthy) + L. Hemisphere of B (healthy) → No LVO
- R. Hemisphere of A (healthy) + R. Hemisphere of B (LVO) → Right LVO
- R. Hemisphere of B (LVO) + R. Hemisphere of C (healthy) → Left LVO

By recombination all desirable LVO cases can be constructed. Even if, hypothetically, the given medical imaging data only consist out of right LVOs, with the recombination method it is possible to construct left LVOs only, no LVOs only, or, as preferred, a balanced distribution of all three. It must be mentioned that the combination of two LVO positive classified hemispheres does not lead to meaningful recombination.

To demonstrate the numbers emerging by recombination, assume medical imaging data of 100 patients' and suppose 50 of those patients suffer an LVO. That means 150 healthy (LVO negative) hemispheres and 50 sick (LVO positive) hemispheres are available. By recombination it would be possible to recombine 50 LVO positive hemispheres with the 150 LVO negative hemispheres (50 x 150 = 7500) and that twice (7500 x 2 = 15000), as left and right can be swapped. The LVO negative hemispheres can be recombined to in total 22350 (150 x 149 = 22350) combinations. All in all, given 100 patients, 37350 datasets can be constructed by the recombination trick. That is an increase in data of 37250%.

In particular, the first dataset and mirrored second dataset for stacking as the training data are chosen based on boundary conditions. In particular, the first channel dataset and the first dataset and/or the mirrored second channel dataset and the mirrored second dataset from the same patient, scan and/or cerebral hemisphere. In particular, for the generation of training data as a first dataset and/or as a first channel dataset an actual mirrored second dataset and/or mirrored second channel dataset can be chosen. For the generation of training data as a mirrored second dataset and/or as a mirrored second channel dataset an actual first dataset and/or first channel dataset can be chosen. The choosing can be implemented by a computer, a processing unit or preferably a machine learned algorithm. The boundary condition is for example chosen to generate training data with a preferred characteristic. Particularly, the boundary conditions chose the datasets based on a comparable amount of contrast agent, patient information data, e.g., age, sex, demography, and/or on characteristics of the cerebral disease.

In a second aspect, the invention also concerns a method for training a deep learning algorithm to detect a disease information, in particular to detect a cerebral disease information, a cerebrovascular disease or an LVO. The method comprises the step of training the deep learning algorithm with training data comprising a first dataset and a mirrored second dataset. The training data are especially generated and/or product of applying the method for generating training data for training a deep learning algorithm on medical imaging data. In this manner, a small amount of available training data in the form of acquired image data can be generated a huge amount of training data, resulting in a better training and higher quality of output information. All features and remarks regarding the method to the training data also apply here. To train the deep learning algorithm output training data are received. The output training data belong and/or are related to the training data. The output training data comprises for example an information regarding the disease, e.g. disease yes/no or no LVO, LVO in first hemisphere or LVO in second hemisphere (mirrored second datasets). The training data {V¹, V_{c}¹, V^{2*}, V_{c}^{2*}} can be combined with the output training data k to a training dataset, e.g. the training dataset {{V¹, V_{c}¹, V^{2*}, V_{c}^{2*}}, k} .

The deep learning algorithm for detection of the disease information can be based on one or more of the following architectures: convolutional neural network, deep belief network, random forest, deep residual learning, deep reinforcement learning, recurrent neural network, Siamese network, generative adversarial network or autoencoder. The deep learning algorithm is trained based on the training data and the output training data, especially based on the training dataset. The training can be done based on federated learning, wherein the medical imaging data or the training data are provided by a first device, e.g., a scanner, and wherein the training of the deep learning algorithm is performed at a second device, e.g., a cloud. The trained deep learning algorithm is provided, especially provided for a computer-implementation or for storage on a storage medium.

In particular, the training of the deep learning algorithm exploits a symmetry of the training data and the related output training data, wherein the symmetry describes the influence of switching of the first dataset and the mirrored second dataset in the training data on the related output training data. Particularly, the stacking of both hemispheres of one or more patients enables new regularization methods, exploiting the symmetry of a prediction. To describe the regularization method designed, notations are introduced. The presence of a cerebral disease information on the first (left) or second (right)hemisphere or not present, in this example LVO or no LVO is indicated by y∈{-1,0 ,1} whereas -1 represents left LVOs, 1 stands for right LVOs and 0 for no LVOs. -1 and 1 are referred to LVO positive and 0 to LVO negative. The two hemispheres of a patient are represented with hₗ=h₁ for the left hemisphere and respectively hᵣ=h₂ for the right hemisphere. A dataset d={s,y} therefore consists of the stack of the hemispheres s={hₗ,hᵣ} and its label y. For every dataset, there exists a mirrored representation d*={s*,-y} with swapped hemispheres s*={hᵣ,hₗ} and labels. With the labels defined above a negation flips the label to left or right but has no effect if no LVO is present. A classifier f(s)=y takes s and returns the prediction *ŷ*. It ideally flips the prediction (in case of LVO and stays at 0 if no LVO) if s* is given instead. This symmetrical behavior can be reinforced and integrated into the training. For instance, latent space feature representations inside a neural network f(g(s))=y defined as g(s)=k must be mirrored to its counterpart g(s* )=k* depending on its label. Contrastive losses for three classes e.g., utilizing the cosine similarity would provide a good fundament for the reinforcement of the desired feature space.

A further aspect of the invention is directed to a method for detecting a disease information, especially detecting a cerebral disease information. Particularly, the disease manifests in anomalies in the tissue, wherein the anomalies can be detected as the disease information. The manifestation of the disease information used for detection based on the medical imaging data is also called radiological finding. The method is applicable detecting diseases with an asymmetric expression in the examination area, particularly, diseases manifesting in one of the two parts of the organ, e.g. in one of the two hemispheres. The method is especially configured for detecting large vessel occlusions, infarct nuclei, penumbras and/or transitions between dead and intact tissue.

Medical imaging data of an examination area of a patient are received, e.g., from a scanner, a data storage or a cloud. The examination area of the patient comprises a first and a second part of a symmetric organ, e.g. a first and a second cerebral hemisphere. The brain atlas data are received. Based on the brain atlas data the medical imaging data are registered into registered imaging data. Channel data of one or more channels can be computed and/or provided for the medical imaging data. In case channel data are provided or computed, it is preferred to register the channel data based on the brain atlas data into registered channel data. The unregistered or registered imaging data, and in particular the unregistered or registered channel data, are split along a symmetry plane or symmetry axis into a first dataset and a second dataset, and in particular a first channel dataset and a second channel dataset. The first dataset, and in particular the first channel dataset, comprises the unregistered or registered imaging data, and in particular the unregistered or registered first channel data of the first part of the organ. The second dataset, in particular the second channel dataset, comprises the unregistered or registered imaging data, and in particular the unregistered or registered channel data, of the second part of the organ. The second dataset, and in particular the second channel dataset, is mirrored along the symmetry plane or symmetry axis. The first dataset and the mirrored second dataset are stacked together to generate subject patient image data. The subject patient image data comprise the stack of the first dataset and the mirrored second dataset. Especially, to generate the subject patient image data the first dataset, the first channel dataset, the mirrored second dataset and the mirrored second channel dataset are stacked. The subject patient image data are preferably constructed, structured and/or assembled as the training data according to the method disclosed above. In particular, the medical imaging data, the registering, the splitting and the mirroring are implemented and/or configured as outlined for the method to generate the training data.

The subject patient image data are analysed using the trained deep learning algorithm. The subject patient image data are input data for the trained deep learning algorithm. The deep learning algorithm is configured to detect, especially to classify and/or localize the disease, especially the disease information.

Preferably, the method comprises the determination of a preferred view information, wherein the preferred view information relates to a radiological finding. The preferred view information comprises and/or defines a projection and/or a preferred projection angle to show and/or display the radiological finding, especially a region of the radiological finding based on the medical imaging data. For shortening, in the following the term preferred view is used synonymous for the best projection and/or best projection angle. Preferred view means especially the view, image, projection and/or projection angle able to display or show the radiological finding to a doctor or person, e.g., for setting a medical diagnosis. The radiological finding is preferably based on the cerebral disease information or the manifestation of the disease in the medical imaging data. The radiological finding is received. The radiological finding is for example made by a doctor or by applying computer implemented method onto medical imaging data. The radiological finding may be provided by a user or a system, e.g., the computer implemented method. The preferred view information is detected based on applying a view determining algorithm on the computed medical image data or on the subject patient image data.

It is noted that this embodiment is also advantageous independently from using the subject patient image data and/or using the trained deep learning algorithm. Thus, a method and system for detecting the cerebral disease information and determining the preferred view information are conceivable. The method would comprise the following steps:
- Receiving medical imaging data of an examination region of subject patient,
- Receiving a radiological finding for the medical imaging data,
- Analyzing the medical imaging data using a view determining algorithm,
- Determining a preferred view information by applying a view determining algorithm on the medical imaging data, wherein the preferred view information comprises a preferred projection and/or a preferred projection angle to show a region of the radiological finding based on the medical imaging data.

This embodiment can be integrated into the visualization software of a system, especially a tomography, as a tool which recommends or automatically chooses the ideal 2D view of the volumetric (3D) segmentation and raw data, such that in said view, the pathology of concern is visible as best as possible. This would ease the diagnosis and shorten the time for it. The main challenge lies in how to obtain suitable labels for what constitutes a good view in order to build a trainable recommender system. In case a trainable system is required for the localization of a disease information, these 3D positions would typically need to be labeled as well, which is a tedious process. The method is especially configured to be implemented in the diagnosis of cerebrovascular diseases using a segmentation of the vessel tree, where it is tedious to provide proper labels. The method is in particular configured to solve the problem of finding the camera position in space which best displays the key indicator for the identification of some disease, and the location of said key characteristic position responsible for the disease.

The method to determine the be preferred view information is based on a multi-view projection approach classifying the disease on 2D views of the segmentation. Preferably, the method uses saliency maps to evaluate the importance of each view.

Related to this problem is the computation of a saliency map on 3D meshes. Song and Liu [Mesh Saliency via Weekly Supervised Classification-for-Saliency CNN", IEEE Transactions on Visualization and Computer Graphics, 2019] presented a method which can compute the saliency map on 3D meshes. Their method uses a multi-view approach and maps the saliency map of these views onto the original 3D surface to visualize the importance of each vertex of the graph of interest. However, their method is restricted to the mesh itself. If the patho-logic sign of interest is the absence of parts of the segmented structure, as it is the case for various vascular diseases like LVOs, indicating saliency on the structure itself is insufficient, hence a different approach is suggested.

The goal of this embodiment is to predict the preferred view information T*, especially the best possible view, defining essentially the extrinsic "camera" parameter to be chosen for the visualization of a disease. According to an embodiment of the invention, a recommender system is applied, here driven by a neural network, which receives a 3D representation and returns T*. To realize this several steps are required as labels for T* are hard to assess, especially as for one case, several ideal views might exist. Instead, the trainable method proposed here, is weakly supervised, wherein labels are used for the case-level classification of the disease to detect, e.g., whether a patient suffers an AVM or not.

Preferably, the method to determine the preferred view information comprises two phases followed by an optional third phase:
Phase 1 is a multi-view classification, wherein a network trains the classification task at hand, where a network classifies whether a patient suffers a particular disease or not. This is required for the view proposal, as the network must learn first to differentiate between positive and negative cases. This is also required as the network needs to learn to extract the descriptive features required to be identified for mentioned diseases. However, the network does not receive the volume as a plain 3D representation, instead it receives arbitrarily many projections N of the volume defined by a set of transformations T = {T₁, T₂,... ,T_{N}} where each transformation has different Euler angles (orientation in space) and/or translation vectors (position in space). Between all cases the various projections are fixed, so the network learns which of them is relevant and which not. The network receives all these projections, for instance in the form of input channels. The multi view classification can be based on the method published by Su et al. ["Multi-view Convolutional Neural Networks for 3D Shape Recognition", Proceedings of ICCV 2015. http://arxiv.org/abs/1505.00880]. However, the classification on the multiple views can be preceding to the method for determining the preferred view information, and just one step out of three required to provide the trained best preferred algorithm.
Phase 2 is for determining the preferred view information. Given a trained multi-view classifier, the goal of phase 2 is to identify the most relevant projection by analysis of the net saliency or relevance of each view. For this, several methods can be chosen, which visualize and quantify the relevance of each input channel. Notable methods include GradCam, Saliency Maps and LRP, just to name a few. Once the most relevant channel has been found, implicitly the preferred view information as the best possible transformation out of the set T = {T₁, T₂,... ,T_{N}} is determined. With the approach, only weak labels are required. Weak labels in this context means that only labels for the first phase are required to train the network for the classification task at hand. Hence, the effort in preparing such a method to determine the preferred view information is manageable. Crucially, the definition of what constitutes an optimal view follows directly from the objective ability (of the trained phase 1 model) to classify the pathology on this view, i.e. it is inherently optimal for the specific clinical decision. In contrast, manual labeling of views, apart from being tedious, would likely be much more subjective.

An optional phase 3 is directed to a coarse localization. In the third phase, the projection can furthermore be used to determine the region of interest in 3D, essentially yielding a coarse localization based solely on a global weak label. To this end, the projections are used for a back-projection, with the accumulated attention values forming a peak in overlapping positions. This is done using all projections in 3D. Alternatively, the peaks can be identified as 2D points in the projection views that define lines (projection rays) in 3D, and the 3D point with the least total distance to all such lines can be marked as the location of interest in the original volume.

The benefit in the usage of the method for determining the preferred view information is manifold. The characteristic view can be used either as thumbnail in the series overview to give the reader a quick grasp about the pathology of a patient or can be visualized alongside with the other standard view when loading the patient. Furthermore, findings are easier to make as a characteristic image has been found and determined already. All of these aspects may help to accelerate the diagnosis. Further benefit is in the third step, especially in the back projection which is performed spatially in terms of image coordinates and allows to identify regions which are not part of the mesh. This is crucial for example in the diagnosis of LVOs, as their key indicator is the occlusion and the consequent absence important subbranches in the vessel tree like Cerebri Media.

In one aspect the invention relates to a system for determining a disease information, comprising an interface to receive subject patient image data. Further comprising an analyzing unit to analyze the subject patient image data received using a trained deep learning algorithm for determining the disease information, wherein the trained deep learning algorithm has been trained with training data to detect the disease information. The system comprises a determining unit to detect the disease information, especially to determine output information regarding the disease information, based on the analysis of the analyzing unit. Further comprising an interface to output the detected disease information, especially to output the output information determined.

In another aspect the invention relates to a computer program product comprising program elements which induce a data processing system to carry out the steps of the method according to one or more of the disclosed aspects, when the program elements are loaded into a memory of the data processing system.

In another aspect the invention relates to a computer-readable medium on which program elements are stored that can be read and executed by the system, in order to perform the steps of the method according to one or more of the disclosed aspects, when the program elements are executed by the system.

Any of the components of the system mentioned herein or any interface between the components of the system can be embodied in form of hardware and/or software. In particular, an interface can be embodied in form of at least one of a PCI-Bus, a USB or a Firewire. In particular, a unit can comprise hardware elements and/or software elements, for example a microprocessor, a field programmable gate array (an acronym is "FPGA") or an application specific integrated circuit (an acronym is "ASIC").

The system can, for example, comprise at least one of a cloud-computing system, a distributed computing system, a computer network, a computer, a tablet computer, a smartphone or the like. The system can comprise hardware and/or software. The hardware can be, for example, a processor system, a memory system and combinations thereof. The hardware can be configurable by the software and/or be operable by the software. Calculations for performing a step of a method and/or for training a machine learning model may be carried out in a processor.

Data, in particular, the medical imaging data, the brain atlas data and the plurality of datasets, can be received, for example, by receiving a signal that carries the data and/or by reading the data from a computer-readable medium. Data, in particular, the cerebral disease information, can be provided, for example, by transmitting a signal that carries the data and/or by writing the data into a computer-readable medium and/or by displaying the data on a display.

The computer program is for example a computer program product comprising another element apart from the computer program. This other element can be hardware, for example a memory device, on which the computer program is stored, a hardware key for using the computer program and the like, and/or software, for example, a documentation or a software key for using the computer program. A computer-readable medium can be embodied as non-permanent main memory (e.g. random-access memory) or as permanent mass storage (e.g. hard disk, USB stick, SD card, solid state disk).

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature.

Reference is made to the fact that the described methods and the described units are merely preferred example embodiments of the invention and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
- Fig. 1: shows a diagram illustrating methods for generating training data, for training of a deep learning algorithm and for detecting a disease information,
- Fig. 2: shows a diagram illustrating a splitting of registered imaging data,
- Fig. 3: shows a diagram illustrating applying of a deep learning algorithm to stacked data,
- Fig. 4: shows a diagram illustrating a system for detecting the disease information.

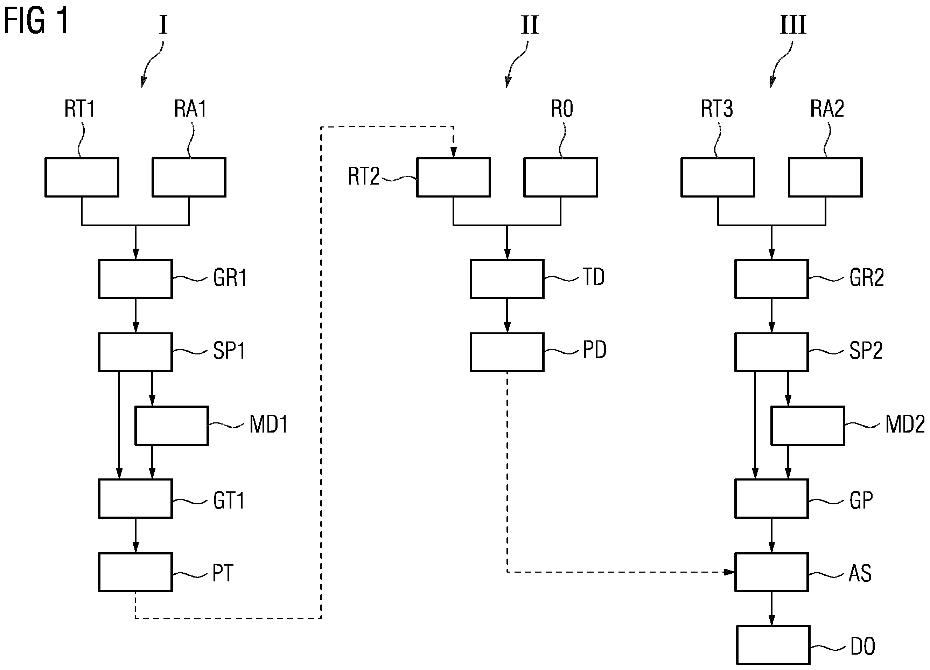

Figure 1 shows a diagram illustrating the combination of three methods I, II and III, wherein method I is for generating training data to train a deep learning algorithm. Method II trains the deep learning algorithm based on the training data. Method III is for determining a disease information based on the trained deep learning algorithm, wherein the disease information according to this example is a cerebral disease information. Furthermore, the example is based on a brain as the symmetric organ, wherein the first part of the organ is a first hemisphere of the brain, and the second part is second hemisphere of the brain.

Method I is configured for generating the training data and comprises:
- Receiving RT1 medical imaging data of an examination area of a patient, the examination area of the patient comprising a first and a second cerebral hemisphere,
- Receiving RA1 brain atlas data,
- Generating GR1 registered imaging data based on the medical imaging data and the brain atlas data,
- Splitting SP1 of the registered imaging data along a symmetry plane or symmetry axis into a first dataset and a second dataset, wherein the first dataset comprises the registered imaging data of the first cerebral hemisphere and the second dataset comprises the registered imaging data of the second cerebral hemisphere,
- Mirroring MD1 of the second dataset along the symmetry plane or symmetry axis,
- Generating GT1 of the training data by stacking of the first dataset and the mirrored second dataset
- Providing PT the training data.

Method II trains the deep learning algorithm and comprises:
- Receiving RT2 training data, wherein the training data are provided by executing method I,
- Receiving RO output training data, wherein the output training data are related to the training data,
- Training TD the deep learning algorithm based on the training data and the output training data,
- Providing PD the deep learning algorithm.

Method III utilizes the trained deep learning algorithm to detect a cerebral disease information and comprises:
- Receiving RT3 medical imaging data of an examination area of a patient, the examination area of the patient comprising a first and a second cerebral hemisphere,
- Receiving RA2 brain atlas data,
- Generating GR2 registered imaging data based on the medical imaging data and the brain atlas data,
- Splitting SP2 of the registered imaging data along a symmetry plane or symmetry axis into a first dataset and a second dataset, wherein the first dataset comprises the registered imaging data of the first cerebral hemisphere and the second dataset comprises the registered imaging data of the second cerebral hemisphere,
- Mirroring MD2 of the second dataset along the symmetry plane or symmetry axis,
- Generating GP of subject patient image data by stacking of the first dataset and the mirrored second dataset
- Analyzing AS the subject patient image data using a trained deep learning algorithm
- Detecting DO the cerebral disease information, wherein the trained deep learning algorithm has been trained with training data.

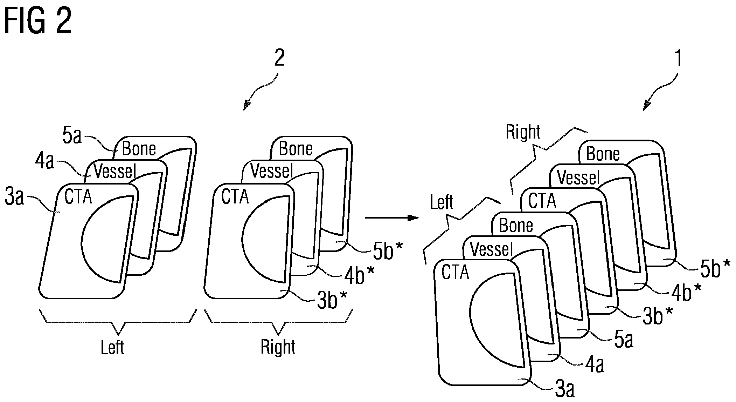

Figure 2 shows an example for generating training data 1 according to an embodiment of the invention. Given is a tomographic scan 2 comprising a CTA dataset of patient as medical imaging data 3. The medical imaging data 3 comprise imaging data of a left cerebral hemisphere as first cerebral hemisphere and imaging data of a right cerebral hemisphere as second cerebral hemisphere. In a first step, additional channels 4, 5 are computed next to the original CTA scan. The channels 4, 5 comprise cannel data of the left hemisphere and cannel data of the right hemisphere. As channel 4 a vessel channel is computed based on segmenting the cerebrovascular vessel tree. As channel 5 a bone channel is computed based on a bone mask. Optionally other channels might be beneficial as well, like a non-contrast CT scan.

In a second step the medical imaging data 3 and the channel data of the channels 4, 5 are registered into a reference coordinate system by registering the volume to a probabilistic atlas of the brain. This standardizes the medical imaging data 3 and the channels 4, 5 to a canonical pose and prepares the dataset for the third step.

In the third step, the medical imaging data 3 and the channel data 4, 5 are split sagittally, such that both hemispheres are separated from each other. One side is flipped to its opposite including all its channels 4, 5. In other words the registered and split medical imaging data of the second cerebral hemisphere are mirrored in the mirrored first dataset 3b*, that visually looks like medical imaging data of a first cerebral hemisphere. Same is done for the channels 4, 5, which results in the mirrored second channel datasets 4b*, 5b*. As a result, we either have visually only left or right sides only. The first dataset 3a, the first channel datasets 4a, 5a, the mirrored second dataset 3b* and the mirrored second datasets 4b*, 4b* are than stacked channel-wise resulting in a 6-channeled volume, whereas the first three channel represent the left hemisphere and the last three channel the right one. Stacking of the data result in the training data 1 configured as a 4D-tensor (3D + 6 Channel). According to this method subject patient image data are generated based on medical imaging data of the patient.

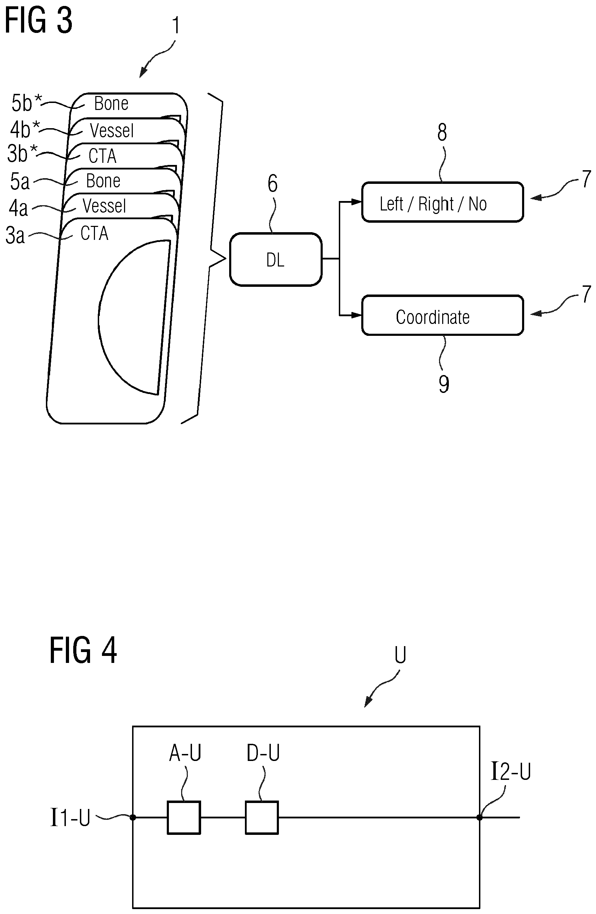

Figure 3 shows an example of applying a deep learning algorithm 6 for determining a cerebral disease information 7 to the training data 1. The deep learning algorithm 6 is configured as a CNN and receives the 6-channeled training data 1 and detects 8 the existence of an LVO and/or localizes 9 it, e.g., by determining the coordinates of the LVO.

Figure 4 shows an example of a system U for detecting a cerebral disease information, comprising an interface I1-U to receive subject patient image data, an analyzing unit A-U to analyze the subject patient image data received using a provided PD trained deep learning algorithm, wherein the trained deep learning algorithm has been trained with provided PT training data to detect the cerebral disease information, a determining unit D-U to determine the output information regarding the cerebral disease information based on analysis of the analyzing unit A-U. Furthermore, the system U comprises a second interface I2-U to output the detected cerebral disease information.

## Claims

1. A method for generating training data (1) for training a deep learning algorithm (6), comprising:
- Receiving (RT1) medical imaging data (2) of an examination area of a patient, the examination area of the patient comprising a first and a second part of a symmetric organ,
- Splitting (SP1) the medical imaging data (2) along a symmetry plane or symmetry axis into a first dataset (3a) and a second dataset (3b), wherein the first dataset (3a) comprises the medical imaging data (2) of the first part of the organ and the second dataset comprises the medical imaging data (2) of the second part of the organ,
- Mirroring (MD1) the second dataset (3b) along the symmetry plane or symmetry axis,
- Generating (GT1) the training data (1) by stacking of the first dataset (3a) and the mirrored second dataset (3b*),
- Providing (PT1) the training data (1).

2. Method according to claim 1, further comprising:
- Receiving (RA1) organ atlas data,
- Generating (GR1) registered imaging data based on the medical imaging data (2) and the organ atlas data,
- Using the registered imaging data as the medical imaging data for the splitting (SP1) into the first dataset (3a) and the second dataset (3b), wherein the first dataset (3a) comprises the registered imaging data (2) of the first part of the organ and the second dataset comprises the registered imaging data (2) of the second part of the organ.

3. Method according to claim 2, wherein the organ atlas data are brain atlas data and the symmetric organ is a brain, wherein the first part of the organ is a first cerebral hemisphere and the second part of the organ is a second cerebral hemisphere.

4. Method according to one of the previous claims, further comprising:
- Receiving at least one channel (4, 5) or calculating at least one channel (4, 5) based on the medical imaging data (2), wherein the channel (4, 5) comprises channel data,
- Generating registered channel data based on the channel data and the organ atlas data,
- Splitting the registered channel data along the symmetry plane or symmetry axis into at least a first channel dataset (4a, 5a) and at least a second channel dataset (4b, 5b), wherein the first channel dataset (4a, 5a) comprises the registered channel data of the first part of the organ and the second channel dataset (4b, 5b) comprises the registered channel data of the second part of the organ,
- Mirroring the second channel dataset (4b, 5b) along the symmetry plane or symmetry axis,
- Generating the training data (1) by stacking of the first dataset (3a), the first channel dataset (4a, 5a), the mirrored second dataset (3b*) and the mirrored second channel dataset (4b*, 5b*).

5. Method according to claim 4, wherein at least one channel (4, 5) is a vessel channel (4) comprising vessel data (4a, 4b) and/or at least one channel (4, 5) is a bone channel (5) comprising bone data (5a, 5b), wherein the vessel data (4a, 4b) are based on a segmentation of a cerebrovascular vessel tree in the medical imaging data (2), wherein the bone data (5a, 5b) are based on a bone mask and/or a segmentation of bones in the medical imaging data (2).

6. Method according to one of the claims 4 or 5, wherein the training data (1) are configured as an ordered set, wherein the first dataset (3a) is prior to the first channel dataset (4a, 4b), the first channel dataset (4a, 4b) is prior to the mirrored second dataset (3b*) and the mirrored second dataset (3b*) is prior to the mirrored second channel dataset (4b*, 5b*).

7. Method according to one of the claims 1 to 6, further comprising:
- Generating (GT1) the training data (1) by stacking of a first dataset (3a) and a mirrored second dataset (3b*) based on medical imaging data (2) of different patients.

8. Method according to claim 7, further comprising:
- Choosing the first dataset (3a) and the mirrored second dataset (3b*) for generating the training data (1) based on boundary conditions, wherein the boundary conditions concern patient information data.

9. A method for training a deep learning algorithm (6) to detect disease information in medical imaging data (2) of an examination area of a patient, comprising:
- Receiving (RT2) training data (1), wherein the training data (1) are generated based on the method according to one of the claims 1 to 8,
- Receiving (RO) output training data, wherein the output training data comprise the disease information of the training data (1),
- Training (TD) the deep learning algorithm (6) based on the training data (1) and the output training data,
- Providing (PD) the trained deep learning algorithm (6).

10. Method according to claim 9, wherein the training of the deep learning algorithm (6) exploits a symmetry of the training data (1) and the output training data, wherein the symmetry describes the influence of switching the first dataset (3a) and the mirrored second dataset (3b*) in the training data (1) on the disease information in the output training data.

11. A method for detecting disease information, comprising:
- Receiving (RT3) medical imaging data (2) of an examination area of a patient, the examination area of the patient comprising a first and a second part of a symmetric organ,
- Splitting (SP2) the medical imaging data (2) along a symmetry plane or symmetry axis into a first dataset (3a) and a second dataset (3b), wherein the first dataset (3a) comprises the medical imaging data of the first part of the organ and the second dataset (3b) comprises the imaging data of the second part of the organ,
- Mirroring (MD2) the second dataset (3b) along the symmetry plane or symmetry axis,
- Generating (GP) subject patient image data by stacking of the first dataset (3a) and the mirrored second dataset (3b*),
- Analyzing (AS) the subject patient image data by applying a trained deep learning algorithm (6) to the subject patient image data,
- Detecting (DO) the disease information based on the analyzing (AS) step, wherein the trained deep learning algorithm (6) has been trained with training data (1), preferably training data (1) according to one of the claims 1 to 8.

12. Method according to claim 11, further comprising:
- Receiving a radiological finding based on the medical imaging data (2) or the subject patient image data,
- Determining a preferred view information by applying a view determining algorithm on the medical imaging data (2) or on the subject patient image data, wherein the preferred view information comprises a preferred projection and/or a preferred projection angle to show a region of the radiological finding based on the medical imaging data (2) or based on the subject patient image data.

13. Method according to claim 12, wherein the medical imaging data (2) comprise 2D-projections from different views onto the region of the radiological finding, further comprising:
- Determining a coarse location of the region of the radiological finding by applying a back-projection on the projections.

14. A system (U) for detecting a disease information, comprising a first interface (I1-U), a processing unit (P-U), an analyzing unit (A-U), a determining unit (D-U) and a second interface (I2-U), wherein:
- the interface (I1-U) is configured to receive medical imaging data (2) of an examination area of a patient, wherein the examination area of the patient comprises a first part and a second part of a symmetric organ,
- wherein the processing unit (P-U) is configured to split the medical imaging data (2) along a symmetry plane or symmetry axis into a first dataset (3a) and a second dataset (3b), wherein the first dataset (3a) comprises the medical imaging data of the first part of the organ and the second dataset (3b) comprises the medical imaging data of the second part of the organ, to mirror the second dataset (3b) along the symmetry plane or symmetry axis and to generate subject patient image data by stacking of the first dataset (3a) and the mirrored second dataset (3b*),
- wherein the analyzing unit (A-U) is configured to analyze the subject patient image data using a trained deep learning algorithm (6) for determining the disease information,
- wherein the determining unit (D-U) is configured to determine output information regarding the disease information based on the analysis of the analyzing unit (A-U),
- wherein the second interface (I2-U) is configured to output the output information.

15. Computer program comprising instructions which, when the program is executed by a computer, cause the computer to carry out at least one of the methods according to one of the claims 1 to 13.
